# EUROPEAN PATENT APPLICATION

(11) **EP 4 729 057 A1**
(43) Date of publication of application: **22.04.2026**
(21) Application number: 23941076.4
(22) Date of filing: 16.06.2023
(51) Int. Cl.: A61K 31/395, A61K 31/4375, A61K 9/06, A61P 31/04

(54) **METHOD OF TREATING DISEASE USING COMPOUND, AND USE OF COMPOUND**

(71) Applicant: TenNor Therapeutics (Suzhou) Limited, Suzhou, Jiangsu 215123 (CN)
(72) Inventor: MA, Zhenkun, Suzhou, Jiangsu 215123 (CN); WANG, Huan, Suzhou, Jiangsu 215123 (CN); HE, Shijie, Suzhou, Jiangsu 215123 (CN); GENG, Guozhu, Suzhou, Jiangsu 215123 (CN); ZHANG, Ling, Suzhou, Jiangsu 215123 (CN)
(74) Representative: Weickmann & Weickmann PartmbB
(86) International application number: PCT/CN2023/100595
(87) International publication number: WO 2024/254841

(57) **Abstract**

Provided in the present application is a method of preventing or treating diabetic foot infection (DFI) or alleviating a DFI-related disorder. The method comprises administering a topical therapeutic agent to a patient in need thereof, wherein the topical therapeutic agent contains a compound as shown in formula (I) and/or a pharmaceutically acceptable salt, hydrate, solvate, prodrug, metabolite or deuterated compound thereof, and the DFI is caused by Gram-positive bacterial infection.

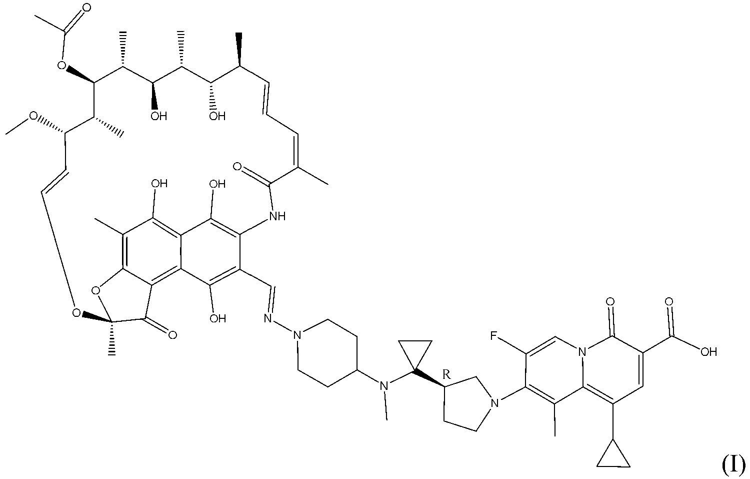

## Description

### TECHNICAL FIELD

The present application relates to the field of biomedicine, and in particular to a method for preventing or treating a disease with a compound, and use of the compound.

### BACKGROUND

Diabetic foot infection (DFI) is one of the serious complications in diabetic patients and a leading cause of lower limb amputations of diabetic patients. Among about 150 million diabetic patients worldwide, 15% to 20% may develop foot ulcers or gangrene during the course of the disease. The amputation rate for patients with a diabetic foot disease is 15 times that for non-diabetic patients, and about 50% of all amputations occur in diabetic patients every year. The treatment of diabetic foot ulcers incurs substantial costs, and the incidence of diabetic foot ulcers in China has shown a significant year-by-year increase. Consequently, the medical and social challenges posed by diabetic foot infections have begun to attract attention from numerous scholars in China. The treatment of diabetic foot infections is often hindered by ischemia, and systemic administration of antibiotics may sometimes only achieve low plasma levels of the antibiotic drugs at the infection site. For example, this issue becomes particularly severe in cases where the infection is caused by drug-resistant bacteria, such as methicillin-resistant *Staphylococcus aureus* (MRSA). Furthermore, currently known medications for treating diabetic foot infections generally involve oral or injectable antibiotics and anti-inflammatory drugs such as cephalosporins, penicillins, and vancomycin. However, these medications have drawbacks, including significant toxic and side effects, a high propensity for inducing drug resistance, the risk of severe allergic reactions in some patients, etc. Therefore, there is an urgent and critical need to improve the treatment of diabetic foot infections, particularly in cases involving specific drug-resistant bacterial infections, and/or patients suffering from severe ischemia, and/or affected bone tissues.

### SUMMARY

In order to address the difficulties in treating diabetic foot infections in the prior art, the present application provides a more practical and effective treatment method.

In one aspect, the present application provides a method for preventing or treating diabetic foot infections (DFI) or alleviating DFI-related disorders, and the method comprises administering a topical therapeutic agent to a patient in need, wherein the topical therapeutic agent comprises a compound represented by formula (I) and/or a pharmaceutically acceptable salt, hydrate, solvate, prodrug, metabolite, or deuteride thereof, and the DFI is caused by Gram-positive bacteria,

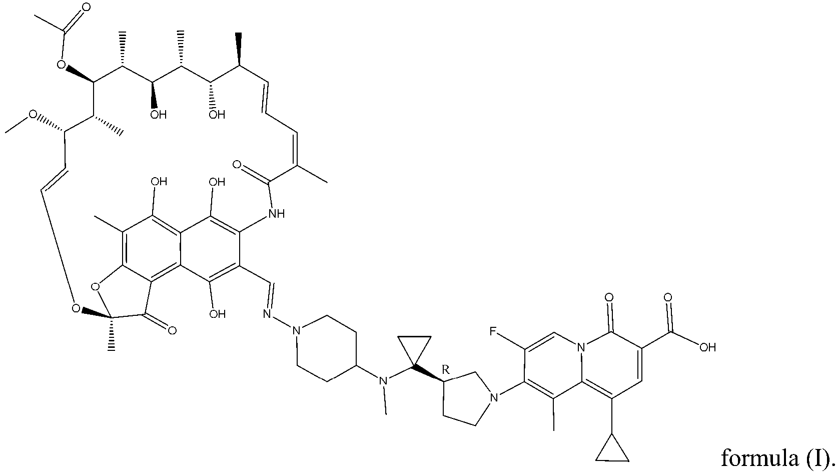

In some embodiments, the Gram-positive bacteria comprise a drug-resistant strain thereof.

In some embodiments, the topical therapeutic agent is an ointment formulation.

In some embodiments, the topical therapeutic agent further comprises one or more of polyethylene glycol 400, polyethylene glycol 3350, propylene glycol, a solubilizer, and an antioxidant.

In some embodiments, the topical therapeutic agent consists of the polyethylene glycol 400, the polyethylene glycol 3350, the propylene glycol, the solubilizer, the antioxidant, and the compound represented by formula (I) and/or the pharmaceutically acceptable salt, hydrate, solvate, prodrug, metabolite, or deuteride thereof.

In some embodiments, the compound represented by formula (I) and/or the pharmaceutically acceptable salt, hydrate, solvate, prodrug, metabolite, or deuteride thereof are used in an amount of about 0.3% to about 0.7% of the total mass of the topical therapeutic agent.

In some embodiments, the polyethylene glycol 400 is used in an amount of about 55% to about 60% of the total mass of the topical therapeutic agent.

In some embodiments, the polyethylene glycol 3350 is used in an amount of about 28% to about 33% of the total mass of the topical therapeutic agent.

In some embodiments, the propylene glycol is used in an amount of about 8% to about 12% of the total mass of the topical therapeutic agent.

In some embodiments, the solubilizer is used in an amount of about 0.1% to about 1% of the total mass.

In some embodiments, the antioxidant is used in an amount of about 0.5% to about 1% of the total mass.

In some embodiments, the topical therapeutic agent consists of the following components: based on a total mass percentage of 100%, the compound represented by formula (I) and/or the pharmaceutically acceptable salt, hydrate, solvate, prodrug, metabolite, or deuteride thereof used in an amount of about 0.3% to about 0.7% of the total mass of the topical therapeutic agent, the polyethylene glycol 400 used in an amount of about 55% to about 60% of the total mass of the topical therapeutic agent, the polyethylene glycol 3350 used in an amount of about 28% to about 33% of the total mass of the topical therapeutic agent, the propylene glycol used in an amount of about 8% to about 12% of the total mass of the topical therapeutic agent, the solubilizer used in an amount of about 0.1% to about 1% of the total mass of the topical therapeutic agent, and the antioxidant used in an amount of about 0.5% to about 1% of the total mass of the topical therapeutic agent.

In some embodiments, the method comprises administering the topical therapeutic agent to the patient in need at an administration frequency optionally selected from the following: once every 3 days, once every 2 days, once daily, and twice daily.

In some embodiments, the diabetic foot infection is caused by *Staphylococcus aureus* and/or coagulase-negative *staphylococcus.*

In some embodiments, the *Staphylococcus aureus* comprises a drug-resistant strain thereof, and the coagulase-negative *staphylococcus* comprises a drug-resistant strain thereof.

In some embodiments, the method further comprises administering an effective amount of an additional therapeutic agent to the patient in need.

In another aspect, the present application provides use of the compound represented by formula (I) and/or the pharmaceutically acceptable salt, hydrate, solvate, prodrug, metabolite, or deuteride thereof in preparing a topical therapeutic agent for preventing or treating diabetic foot infections (DFI) or alleviating DFI-related disorders, wherein the DFI is caused by Gram-positive bacteria, and the topical therapeutic agent comprises the compound represented by formula (I) and/or the pharmaceutically acceptable salt, hydrate, solvate, prodrug, metabolite, or deuteride thereof.

In some embodiments, the Gram-positive bacteria comprise the drug-resistant strain thereof.

In some embodiments, the topical therapeutic agent is an ointment formulation.

In some embodiments, the topical therapeutic agent further comprises one or more of polyethylene glycol 400, polyethylene glycol 3350, propylene glycol, a solubilizer, and an antioxidant.

In some embodiments, the topical therapeutic agent consists of the polyethylene glycol 400, the polyethylene glycol 3350, the propylene glycol, the solubilizer, the antioxidant, and the compound represented by formula (I) and/or the pharmaceutically acceptable salt, hydrate, solvate, prodrug, metabolite, or deuteride thereof.

In some embodiments, the compound represented by formula (I) and/or the pharmaceutically acceptable salt, hydrate, solvate, prodrug, metabolite, or deuteride thereof are used in an amount of about 0.3% to about 0.7% of the total mass of the topical therapeutic agent.

In some embodiments, the polyethylene glycol 400 is used in an amount of about 55% to about 60% of the total mass of the topical therapeutic agent.

In some embodiments, the polyethylene glycol 3350 is used in an amount of about 28% to about 33% of the total mass of the topical therapeutic agent.

In some embodiments, the propylene glycol is used in an amount of about 8% to about 12% of the total mass of the topical therapeutic agent.

In some embodiments, the solubilizer is used in an amount of about 0.1% to about 1% of the total mass.

In some embodiments, the antioxidant is used in an amount of about 0.5% to about 1% of the total mass.

In some embodiments, the topical therapeutic agent consists of the following components: based on a total mass percentage of 100%, the compound represented by formula (I) and/or the pharmaceutically acceptable salt, hydrate, solvate, prodrug, metabolite, or deuteride thereof used in an amount of about 0.3% to about 0.7% of the total mass of the topical therapeutic agent, the polyethylene glycol 400 used in an amount of about 55% to about 60% of the total mass of the topical therapeutic agent, the polyethylene glycol 3350 used in an amount of about 28% to about 33% of the total mass of the topical therapeutic agent, the propylene glycol used in an amount of about 8% to about 12% of the total mass of the topical therapeutic agent, the solubilizer used in an amount of about 0.1% to about 1% of the total mass of the topical therapeutic agent, and the antioxidant used in an amount of about 0.5% to about 1% of the total mass of the topical therapeutic agent.

In some embodiments, the topical therapeutic agent is formulated to be suitable for administration to a patient in need at an administration frequency optionally selected from the following: once every 3 days, once every 2 days, once daily, and twice daily.

In some embodiments, the diabetic foot infection is caused by *Staphylococcus aureus* and/or coagulase-negative *staphylococcus.*

In some embodiments, the *Staphylococcus aureus* comprises a drug-resistant strain thereof, and the coagulase-negative *staphylococcus* comprises a drug-resistant strain thereof.

In some embodiments, the topical therapeutic agent is formulated to be suitable for co-administration with an effective amount of an additional therapeutic agent to the patient in need.

### DETAILED DESCRIPTION

The implementation of the invention of the present application is described below with reference to specific embodiments, and other advantages and effects of the invention of the present application will be readily apparent to those skilled in the art from the disclosure of the specification.

### Definitions of Terms

In the present application, the term "patient in need" generally may include human patients and other mammalian subjects that receive prophylactic or therapeutic treatment, including but not limited to non-human primates, laboratory animals such as rabbits, dogs, rats, and mice, as well as other animals. These "patients in need" may illustratively be subjects with a diagnosed specific disease, subjects who are receiving treatment related to a specific disease, subjects who are at a tendency or risk of developing a specific disease, or the like.

In the present application, the term "administer", "administration", or "administering" generally refers to introducing the drug or a composition comprising the drug (*e.g.,* the therapeutic agent of the present application) into the body of a patient by any route of introduction or delivery. Any method for contacting a cell, organ, or tissue with the drug or the composition thereof known to those skilled in the art can be employed.

In the present application, the term "therapeutic agent" generally refers to a compound, a mixture of compounds, a composition, a biomacromolecule, or an extract made from biological materials that, when properly administered to a patient, is capable of inducing a desired therapeutic effect, preventing the development of a certain disorder, or alleviating an associated disorder.

In the present application, the term "pharmaceutically acceptable salt" generally refers to a conventional acid- or base-addition salt that retains the biological efficacy and properties of the parent compound (*e.g.*, the compound represented by formula (I)). By way of example only, acid-addition salts may be formed from suitable non-toxic organic or inorganic acids. Exemplary acid-addition salts include those derived from inorganic acids such as hydrochloric acid, hydrobromic acid, hydroiodic acid, sulphuric acid, sulfamic acid, phosphoric acid, and nitric acid, and those derived from organic acids such as p-toluenesulfonic acid, naphthalenesulfonic acid, naphthalenedisulfonic acid, methanesulfonic acid, ethanesulfonic acid, and trifluoroacetic acid; base-addition salts include those derived from inorganic bases such as sodium salts, potassium salts, lithium salts, ammonium salts, calcium salts, and magnesium salts, and those derived from organic bases such as salts of primary amine, secondary amine, and tertiary amine. The chemical modification of (drug) compounds into salts is a well-known technique for medicinal chemists to achieve improved physical and chemical stability, hygroscopicity, flowability, and solubility of the compounds. See, *e.g.,* Ansel, H., et al., Pharmaceutical Dosage Forms and Drug Delivery Systems, 6th ed., 1995.

In the present application, the term "solvate" generally refers to a coordination complex or complex of one or more solvent molecules with the salt form of the compound of the present application.

Examples of the solvents for forming solvates include, but are not limited to, water, isopropanol, ethanol, methanol, DMSO, ethyl acetate, acetic acid, and ethanolamine. The term "hydrate" generally refers to a complex in which the solvent molecule is water.

In the present application, the term "prodrug" generally refers to that the prodrug substance may be administered to a patient and then metabolized to form a substance with the compound structure of the present application; the term "metabolite" generally refers to a substance obtained by administration of the compound of the present application to a patient and metabolism. Prodrugs and metabolites encompassed in such derivatives are included within the scope of the invention of the present application.

In the present application, the terms "deuteride" and "deuterated compound" are used interchangeably and generally refer to a novel compound obtained by substituting one or more hydrogen atoms in an organic compound with deuterium atoms.

In the present application, the term "ointment formulation" generally refers to a homogeneous semisolid topical formulation prepared by mixing a drug with an oleaginous base or a water-soluble base.

Due to the different dispersion states of the drug in the base, ointment formulations may be classified into solution-type ointments and suspension-type ointments. The solution-type ointments are ointment dosage forms prepared by dissolving or co-dissolving the drug in the base or base components, while the suspension-type ointments are ointment dosage forms prepared by uniformly dispersing the fine drug powder in the base. The ointment formulations provided by the present application are generally used in topical and local treatment, and in certain embodiments, the drug (*e.g.,* the compound of formula (I) described in the present application) in the ointment formulations can exert a systemic pharmacological or therapeutic effect upon transdermal absorption. In certain embodiments, the base of the ointment dosage form serves as an excipient and a pharmaceutical carrier, which can have an important influence on the quality of the ointment and the release and absorption of the drug. The bases of the ointment dosage forms commonly used in the art can be classified into oleaginous bases, emulsion bases, and water-soluble bases. The ointment dosage forms required for the treatment method of the present application may be obtained using suitable preparation methods and processes in the art.

In the present application, the term "selected from" generally refers to the inclusion of the selected objects and all combinations thereof. For example, "selected from (:) A, B, and C" refers to the inclusion of all combinations of A, B, and C, *e.g.,* A, B, C, A+B, A+C, B+C, or A+B+C.

In the present application, the terms "effective amount" and "effective dose" can be used interchangeably and generally refer to an amount sufficient to achieve, or at least partially achieve, the desired therapeutic effect. When used for a patient in need, it generally refers to an amount sufficient to cure an existing diseased patient or at least partially prevent the disease and complications thereof. The effective amount for this use will depend on the severity of the patient's infection and the overall state of the patient's own immune system.

In the present application, the term "co-administration" generally refers to the administration of the therapeutic agent described in the present application together with another therapeutic agent or drug, either as a single formulation or as multiple independent formulations. The co-administration may be carried out simultaneously or sequentially in any order, *e.g*., preferably for a certain period of time while both (or all) pharmaceutically active ingredients simultaneously exert their biological activities.

The therapeutic agent of the present application and the another therapeutic agent are not necessarily administered by the same route of administration.

In the present application, the term "about" or "approximately" generally means being within an acceptable error range for a particular value as determined by those of ordinary skills in the art, which depends in part on how the value is measured or determined, *i.e.,* the limitations of the measurement system. For example, "about" or "approximately" may mean being within 1 or more than 1 standard deviation in accordance with the practice in the art. Alternatively, "about" or "approximately" may mean a range of up to 10% or 20% (*i.e.,* ±10% or ±20%). For example, about 3 mg may include any number between 2.7 mg and 3.3 mg (for 10%) or between 2.4 mg and 3.6 mg (for 20%). Furthermore, particularly for biological systems or processes, the term may mean a value up to an order of magnitude or up to 5 fold. Unless otherwise specified, when a specific value or composition is provided in the specification and the appended claims, the meaning of "about" or "approximately" should be assumed to be within an acceptable error range for the specific value or composition.

### Detailed Description of the Invention

In one aspect, the present application provides a method for preventing or treating diabetic foot infections (DFI) or alleviating DFI-related disorders, and the method comprises administering a topical therapeutic agent to a patient in need, wherein the topical therapeutic agent comprises a compound represented by formula (I) and/or a pharmaceutically acceptable salt, hydrate, solvate, prodrug, metabolite, or deuteride thereof, and the DFI is caused by Gram-positive bacteria. In some embodiments, the Gram-positive bacteria may comprise a drug-resistant strain thereof.

In some embodiments, the diabetic foot infections are caused by *Staphylococcus aureus* and/or coagulase-negative *staphylococcus.* For example, the *Staphylococcus aureus* may further comprise a drug-resistant strain thereof, and the coagulase-negative *staphylococcus* may further comprise a drug-resistant strain thereof. Specifically, the coagulase-negative *Staphylococcus* may be selected from *Staphylococcus epidermidis, Staphylococcus saprophyticus, Staphylococcus xylosus, Staphylococcus haemolyticus, Staphylococcus capitis, Staphylococcus warneri, Staphylococcus simulans, Staphylococcus xylosus subsp. xylosus,* and *Staphylococcus simiae.* In the present application, the drug-resistant strain may demonstrate single-drug resistance to rifamycin (*e.g*., rifampicin), single-drug resistance to quinolone or quinolizinone, or resistance to both rifamycin and quinolizinone. For example, the drug-resistant strain may also be resistant to other antibiotics, including macrolides such as clarithromycin, azithromycin, and roxithromycin; nitroimidazoles such as metronidazole, ornidazole, pretomanid, and delamanid; aminoglycosides such as streptomycin and amikacin; β-lactams such as methicillin, ampicillin, and amoxicillin; tetracyclines such as tetracycline, tigecycline, and minocycline; oxazolidinones such as linezolid and tedizolid; nitrofurans such as furazolidone; polypeptides such as vancomycin and polymyxin; diarylquinolines such as bedaquiline; and clofazimine.

In some embodiments, the topical therapeutic agent may be an ointment formulation.

In some embodiments, the topical therapeutic agent further comprises one or more of polyethylene glycol 400, polyethylene glycol 3350, propylene glycol, a solubilizer, and an antioxidant. For example, the topical therapeutic agent consists of the polyethylene glycol 400, the polyethylene glycol 3350, the propylene glycol, the solubilizer, the antioxidant, and the compound represented by formula (I) and/or the pharmaceutically acceptable salt, hydrate, solvate, prodrug, metabolite, or deuteride thereof. Preferably, the solubilizer described in the present application may include triethanolamine. Preferably, the antioxidant described in the present application may include vitamin C, vitamin E, or a combination thereof.

In some embodiments, the compound represented by formula (I) and/or the pharmaceutically acceptable salt, hydrate, solvate, prodrug, metabolite, or deuteride thereof are used in an amount of about 0.3% to about 0.7% of the total mass of the topical therapeutic agent. For example, the compound represented by formula (I) and/or the pharmaceutically acceptable salt, hydrate, solvate, prodrug, metabolite, or deuteride thereof are used in an amount of about 0.30%, about 0.35%, about 0.40%, about 0.45%, about 0.50%, about 0.55%, about 0.60%, about 0.65%, or about 0.70% of the total mass of the topical therapeutic agent.

In some embodiments, the polyethylene glycol 400 is used in an amount of about 55% to about 60% of the total mass of the topical therapeutic agent. For example, the polyethylene glycol 400 is used in an amount of about 55%, about 56%, about 57%, about 58%, about 59%, or about 60% of the total mass of the topical therapeutic agent.

In some embodiments, the polyethylene glycol 3350 is used in an amount of about 28% to about 33% of the total mass of the topical therapeutic agent. For example, the polyethylene glycol 3350 is used in an amount of about 28%, about 29%, about 30%, about 31%, about 32%, or about 33% of the total mass of the topical therapeutic agent.

In some embodiments, the propylene glycol is used in an amount of about 8% to about 12% of the total mass of the topical therapeutic agent. For example, the propylene glycol is used in an amount of about 8.0%, about 8.5%, about 9.0%, about 9.5%, about 10.0%, about 10.5%, about 11.0%, about 11.5%, or about 12.0% of the total mass of the topical therapeutic agent.

In some embodiments, the solubilizer is used in an amount of about 0.1% to about 1% of the total mass. For example, the solubilizer is used in an amount of about 0.10%, about 0.15%, about 0.20%, about 0.25%, about 0.30%, about 0.35%, about 0.40%, about 0.45%, about 0.50%, about 0.55%, about 0.60%, about 0.65%, about 0.70%, about 0.75%, about 0.80%, about 0.85%, about 0.90%, about 0.95%, or about 1.00% of the total mass.

In some embodiments, the antioxidant is used in an amount of about 0.5% to about 1% of the total mass. For example, the antioxidant is used in an amount of about 0.50%, about 0.55%, about 0.60%, about 0.65%, about 0.70%, about 0.75%, about 0.80%, about 0.85%, about 0.90%, about 0.95%, or about 1.00% of the total mass.

Preferably, in some embodiments, the topical therapeutic agent consists of the following components: based on a total mass percentage of 100%, the compound represented by formula (I) and/or the pharmaceutically acceptable salt, hydrate, solvate, prodrug, metabolite, or deuteride thereof used in an amount of about 0.3% to about 0.7% (*e.g.,* about 0.30%, about 0.35%, about 0.40%, about 0.45%, about 0.50%, about 0.55%, about 0.60%, about 0.65%, or about 0.70%) of the total mass of the topical therapeutic agent, the polyethylene glycol 400 used in an amount of about 55% to about 60% (*e.g.,* about 55%, about 56%, about 57%, about 58%, about 59%, or about 60%) of the total mass of the topical therapeutic agent, the polyethylene glycol 3350 used in an amount of about 28% to about 33% (*e.g.*, about 28%, about 29%, about 30%, about 31%, about 32%, or about 33%) of the total mass of the topical therapeutic agent, the propylene glycol used in an amount of about 8% to about 12% (*e.g.,* about 8.0%, about 8.5%, about 9.0%, about 9.5%, about 10.0%, about 10.5%, about 11.0%, about 11.5%, or about 12.0%) of the total mass of the topical therapeutic agent, the solubilizer used in an amount of about 0.1% to about 1% (*e.g*., about 0.10%, about 0.15%, about 0.20%, about 0.25%, about 0.30%, about 0.35%, about 0.40%, about 0.45%, about 0.50%, about 0.55%, about 0.60%, about 0.65%, about 0.70%, about 0.75%, about 0.80%, about 0.85%, about 0.90%, about 0.95%, or about 1.00%) of the total mass of the topical therapeutic agent, and the antioxidant used in an amount of about 0.5% to about 1% (*e.g*., about 0.50%, about 0.55%, about 0.60%, about 0.65%, about 0.70%, about 0.75%, about 0.80%, about 0.85%, about 0.90%, about 0.95%, or about 1.00%) of the total mass of the topical therapeutic agent.

In some embodiments, the method comprises administering the topical therapeutic agent to the patient in need at an administration frequency optionally selected from the following: once every 3 days, once every 2 days, once daily, and twice daily.

In some embodiments, the method further comprises administering an effective amount of an additional therapeutic agent to the patient in need. For example, the method further comprises administering an effective amount of another therapeutic agent to the patient by injection administration (*e.g*., intravenous injection, intramuscular injection, and/or subcutaneous injection), oral administration, intracavitary administration, enteral administration, and/or transdermal absorption. For example, the another therapeutic agent may be selected from an antibacterial agent, an anesthetic, an analgesic, a sedative, a hormone drug, an anti-inflammatory drug, an anticoagulant, a platelet aggregation inhibitor, an antipyretic, a cell membrane permeabilizer, a muscle relaxant, and the like.

In another aspect, the present application provides use of the compound represented by formula (I) and/or the pharmaceutically acceptable salt, hydrate, solvate, prodrug, metabolite, or deuteride thereof in preparing a topical therapeutic agent for preventing or treating diabetic foot infections (DFI) or alleviating DFI-related disorders, wherein the DFI is caused by Gram-positive bacteria, and the topical therapeutic agent comprises the compound represented by formula (I) and/or the pharmaceutically acceptable salt, hydrate, solvate, prodrug, metabolite, or deuteride thereof. In some embodiments, the Gram-positive bacteria comprise a drug-resistant strain thereof.

In some embodiments, the diabetic foot infections are caused by *Staphylococcus aureus* and/or coagulase-negative *staphylococcus.* For example, the *Staphylococcus aureus* may further comprise a drug-resistant strain thereof, and the coagulase-negative *staphylococcus* may further comprise a drug-resistant strain thereof. Specifically, the coagulase-negative *staphylococcus* may be selected from *Staphylococcus epidermidis, Staphylococcus saprophyticus, Staphylococcus xylosus, Staphylococcus haemolyticus, Staphylococcus capitis, Staphylococcus warneri, Staphylococcus simulans, Staphylococcus xylosus subsp. xylosus,* and *Staphylococcus simiae.* In the present application, the drug-resistant strain may demonstrate single-drug resistance to rifamycin (*e.g*., rifampicin), single-drug resistance to quinolone or quinolizinone, or resistance to both rifamycin and quinolizinone. For example, the drug-resistant strain may also be resistant to other antibiotics, including macrolides such as clarithromycin, azithromycin, and roxithromycin; nitroimidazoles such as metronidazole, ornidazole, pretomanid, and delamanid; aminoglycosides such as streptomycin and amikacin; β-lactams such as methicillin, ampicillin, and amoxicillin; tetracyclines such as tetracycline, tigecycline, and minocycline; oxazolidinones such as linezolid and tedizolid; nitrofurans such as furazolidone; polypeptides such as vancomycin and polymyxin; diarylquinolines such as bedaquiline; and clofazimine.

In some embodiments, the topical therapeutic agent may be an ointment formulation.

In some embodiments, the topical therapeutic agent further comprises one or more of polyethylene glycol 400, polyethylene glycol 3350, propylene glycol, a solubilizer, and an antioxidant. For example, the topical therapeutic agent consists of the polyethylene glycol 400, the polyethylene glycol 3350, the propylene glycol, the solubilizer, the antioxidant, and the compound represented by formula (I) and/or the pharmaceutically acceptable salt, hydrate, solvate, prodrug, metabolite, or deuteride thereof. Preferably, the solubilizer described in the present application may include triethanolamine. Preferably, the antioxidant described in the present application may include vitamin C, vitamin E, or a combination thereof.

In some embodiments, the compound represented by formula (I) and/or the pharmaceutically acceptable salt, hydrate, solvate, prodrug, metabolite, or deuteride thereof are used in an amount of about 0.3% to about 0.7% of the total mass of the topical therapeutic agent. For example, the compound represented by formula (I) and/or the pharmaceutically acceptable salt, hydrate, solvate, prodrug, metabolite, or deuteride thereof are used in an amount of about 0.30%, about 0.35%, about 0.40%, about 0.45%, about 0.50%, about 0.55%, about 0.60%, about 0.65%, or about 0.70% of the total mass of the topical therapeutic agent.

In some embodiments, the polyethylene glycol 400 is used in an amount of about 55% to about 60% of the total mass of the topical therapeutic agent. For example, the polyethylene glycol 400 is used in an amount of about 55%, about 56%, about 57%, about 58%, about 59%, or about 60% of the total mass of the topical therapeutic agent.

In some embodiments, the polyethylene glycol 3350 is used in an amount of about 28% to about 33% of the total mass of the topical therapeutic agent. For example, the polyethylene glycol 3350 is used in an amount of about 28%, about 29%, about 30%, about 31%, about 32%, or about 33% of the total mass of the topical therapeutic agent.

In some embodiments, the propylene glycol is used in an amount of about 8% to about 12% of the total mass of the topical therapeutic agent. For example, the propylene glycol is used in an amount of about 8.0%, about 8.5%, about 9.0%, about 9.5%, about 10.0%, about 10.5%, about 11.0%, about 11.5%, or about 12.0% of the total mass of the topical therapeutic agent.

In some embodiments, the solubilizer is used in an amount of about 0.1% to about 1% of the total mass. For example, the solubilizer is used in an amount of about 0.10%, about 0.15%, about 0.20%, about 0.25%, about 0.30%, about 0.35%, about 0.40%, about 0.45%, about 0.50%, about 0.55%, about 0.60%, about 0.65%, about 0.70%, about 0.75%, about 0.80%, about 0.85%, about 0.90%, about 0.95%, or about 1.00% of the total mass.

In some embodiments, the antioxidant is used in an amount of about 0.5% to about 1% of the total mass. For example, the antioxidant is used in an amount of about 0.50%, about 0.55%, about 0.60%, about 0.65%, about 0.70%, about 0.75%, about 0.80%, about 0.85%, about 0.90%, about 0.95%, or about 1.00% of the total mass.

Preferably, in some embodiments, the topical therapeutic agent consists of the following components: based on a total mass percentage of 100%, the compound represented by formula (I) and/or the pharmaceutically acceptable salt, hydrate, solvate, prodrug, metabolite, or deuteride thereof used in an amount of about 0.3% to about 0.7% (e.g., about 0.30%, about 0.35%, about 0.40%, about 0.45%, about 0.50%, about 0.55%, about 0.60%, about 0.65%, or about 0.70%) of the total mass of the topical therapeutic agent, the polyethylene glycol 400 used in an amount of about 55% to about 60% (e.g., about 55%, about 56%, about 57%, about 58%, about 59%, or about 60%) of the total mass of the topical therapeutic agent, the polyethylene glycol 3350 used in an amount of about 28% to about 33% (e.g., about 28%, about 29%, about 30%, about 31%, about 32%, or about 33%) of the total mass of the topical therapeutic agent, the propylene glycol used in an amount of about 8% to about 12% (e.g., about 8.0%, about 8.5%, about 9.0%, about 9.5%, about 10.0%, about 10.5%, about 11.0%, about 11.5%, or about 12.0%) of the total mass of the topical therapeutic agent, the solubilizer used in an amount of about 0.1% to about 1% (e.g., about 0.10%, about 0.15%, about 0.20%, about 0.25%, about 0.30%, about 0.35%, about 0.40%, about 0.45%, about 0.50%, about 0.55%, about 0.60%, about 0.65%, about 0.70%, about 0.75%, about 0.80%, about 0.85%, about 0.90%, about 0.95%, or about 1.00%) of the total mass of the topical therapeutic agent, and the antioxidant used in an amount of about 0.5% to about 1% (e.g., about 0.50%, about 0.55%, about 0.60%, about 0.65%, about 0.70%, about 0.75%, about 0.80%, about 0.85%, about 0.90%, about 0.95%, or about 1.00%) of the total mass of the topical therapeutic agent.

In some embodiments, the topical therapeutic agent is formulated to be suitable for administration to a patient in need at an administration frequency optionally selected from the following: once every 3 days, once every 2 days, once daily, and twice daily.

In some embodiments, the topical therapeutic agent is formulated to be suitable for co-administration with an effective amount of an additional therapeutic agent to the patient in need. For example, the topical therapeutic agent may be formulated to be suitable for co-administration to the patient in need with an effective amount of another therapeutic agent, wherein the another therapeutic agent may be administered to the patient by injection administration (*e.g.*, intravenous injection, intramuscular injection, and/or subcutaneous injection), oral administration, intracavitary administration, enteral administration, and/or transdermal absorption. For example, the another therapeutic agent may be selected from an antibacterial agent, an anesthetic, an analgesic, a sedative, a hormone drug, an anti-inflammatory drug, an anticoagulant, a platelet aggregation inhibitor, an antipyretic, a cell membrane permeabilizer, a muscle relaxant, and the like.

Without being bound by any theory, the following examples are intended only to illustrate the methods, use, *etc.,* of the present application, but not to limit the scope of the present application.

### Examples

### Example 1

### Determination of Biofilm Antibacterial Activity of Compound of Formula (I) Against Clinical Isolates from Diabetic Foot Infections (DFI)

### 1. Experimental method

Preparation of test strains: In this example, foot specimens were collected from 49 patients with type II diabetes. Isolates were obtained from the specimens described above through homogenization, plating, and culture at 37 °C, and after the removal of fungi and strains with poor growth or strains that could not be isolated and purified, a total of 69 clinical strains were obtained. Among these, 55 strains were Gram-positive bacteria and 14 strains were Gram-negative bacteria. Of the Gram-positive bacteria, 43 strains were *staphylococci,* including 18 *Staphylococcus aureus* strains, 8 *Staphylococcus epidermidis* strains, and 5 *Staphylococcus lugdunensis* strains. One ATCC standard strain was additionally used as a standard and quality control strain.

Preparation of biofilms: In a sterile environment, a single, identified, and thawed colony was picked with an inoculation loop and bacterial inoculums were adjusted to a 0.5 McFarland standard, which served as an initial bacterial inoculum. The bacterial inoculum was diluted in a 1:200 ratio by adding 50 µL of the initial bacterial inoculums to 10 mL of a CAMHB medium. 150 µL of the bacterial inoculums was transferred to a CBD plate using a pipette. The inoculated CBD plate was incubated in an incubator at 35±2 °C for 16-20 hours.

Determination of antibacterial activity: The minimum inhibitory concentration (MIC) and the minimum biofilm bactericidal concentration (MBBC) for *in vitro* biofilms of the compound of formula (I) and control drugs (see details in 2) against 69 clinically isolated diabetic foot infection clinical strains were evaluated using the standard broth dilution method and the *in vitro* biofilm culture and drug susceptibility methods reported in the literature.

### 2. Analysis of results

**Table 1-1. Summary of the results for the MIC and the MBBC (µg/mL) for biofilms of the compound of formula (I) and control drugs against clinical DFI isolates (Gram-positive bacteria vs. Gram-negative bacteria)**

| **Test drug** | **Gram-positive bacteria (55 strains)** | | | | **Gram-negative bacteria (14 strains)** | | | |
|---|---|---|---|---|---|---|---|---|
| | **MIC₅₀** | **MIC₉₀** | **MBBC₅₀** | **MBBC₉₀** | **MIC₅₀** | **MIC₉₀** | **MBBC₅₀** | **MBBC₉₀** |
| Compound of formula (I) | ≤0.015 | 0.25 | 0.125 | 16 | >32 | >32 | >16 | >32 |
| Rifampicin | ≤0.015 | 0.5 | 8 | >16 | 32 | >32 | >16 | >32 |
| Ciprofloxacin | 0.5 | 32 | 64 | >128 | 0.125 | 2 | 32 | >128 |
| Mupirocin | 0.25 | 64 | >128 | >128 | >128 | >128 | >128 | >128 |
| Clindamycin | 0.25 | >128 | >128 | >128 | >128 | >128 | >128 | >128 |
| Linezolid | 2 | 4 | >128 | >128 | >128 | >128 | >128 | >128 |
| Oxacillin | 2 | 128 | >128 | >128 | >128 | >128 | >128 | >128 |
| Vancomycin | 2 | 2 | >128 | >128 | >128 | >128 | >128 | >128 |

The results in Table 1-1 show that the compound of formula (I) exhibited a significantly superior antibacterial activity against Gram-positive bacterial isolates to that against Gram-negative bacteria: (1) the MIC₅₀ and MIC₉₀ of the compound were ≤0.015 µg/mL and 0.25 µg/mL, respectively, and the compound was significantly superior to the clinical first-line drugs linezolid (MIC₅₀ and MIC₉₀ were 2 µg/mL and 4 µg/mL, respectively) and vancomycin (MIC₅₀ and MIC₉₀ were both 2 µg/mL); (2) the MBBC results of the compound of formula (I) also demonstrate that the compound had a better bactericidal activity against the *in vitro* biofilms of Gram-positive bacteria than that against the *in vitro* biofilms of Gram-negative bacteria, with MBBC₅₀ and MBBC₉₀ being 0.125 µg/mL and 16 µg/mL, respectively, and it was significantly superior to the clinical first-line drugs linezolid (MBBC₅₀ and MBBC₉₀ were both >128 µg/mL) and vancomycin (MBBC₅₀ and MBBC₉₀ were both >128 µg/mL).

**Table 1-2. Summary of the results for the MIC (µg/mL) of the compound of formula (I) and control drugs against clinical DFI isolates (representative Gram-positive bacteria)**

| **Test drug** | **Staphylococci (43 strains)** | | | | | | |
|---|---|---|---|---|---|---|---|
| | **Total** | | | ***Staphylococcus aureus* (18 strains)** | | | ***Staphylococcus epidermidis* (8 strains)** |
| | **MIC range** | **MIC₅₀** | **MIC₉₀** | **MIC range** | **MIC₅₀** | **MIC₉₀** | **MIC range** |
| Compound of formula (I) | ≤0.015 ~ 0.25 | ≤0.015 | ≤0.015 | ≤0.015 ~ 0.03 | ≤0.015 | ≤0.015 | ≤0.015 ~ 0.03 |
| Rifampicin | ≤0.015~ 0.125 | ≤0.015 | ≤0.015 | ≤0.015 | ≤0.015 | ≤0.015 | ≤0.015 |
| Ciprofloxacin | 0.125 ~ >128 | 0.5 | 32 | 0.125 ~ 16 | 0.5 | 2 | 0.25 ~ >128 |
| Mupirocin | ≤0.06 ~ >128 | 0.25 | 32 | 0.25 ~ >128 | 0.25 | >128 | 0.25 ~ 0.5 |
| Clindamycin | ≤0.06 ~ >128 | 0.125 | 128 | ≤0.06 ~ 0.5 | 0.125 | 0.25 | ≤0.06 ~ >128 |
| Linezolid | 0.5 ~ 4 | 2 | 4 | 2 ~ 4 | 2 | 4 | 2 ~ 4 |
| Oxacillin | 0.25 ~ >128 | 2 | 128 | 0.25 ~ 128 | 0.5 | 64 | 0.25 ~ 128 |
| Vancomycin | 1 ~ 4 | 2 | 2 | 1 ~ 2 | 2 | 2 | 2 ~ 4 |

**Table 1-3. Summary of the results for the MBBC (µg/mL) of the compound of formula (I) and control drugs against biofilms of clinical DFI isolates (representative Gram-positive bacteria)**

| | | | | | | | |
|---|---|---|---|---|---|---|---|
| **Test drug** | | | **Staphylococci (43 strains)** | | | | |
| | | | **Total** | ***Staphylococcus aureus* (18 strains)** | | ***Staphylococc us epidermidis* (8 strains)** | |

| | **MBBC range** | **MBBC₅₀** | **MBBC₉₀** | **MBBC range** | **MBBC₅₀** | **MBBC₉₀** | **MBBC range** |
|---|---|---|---|---|---|---|---|
| Compound of formula (I) | ≤0.015 ~ 1 | 0.06 | 0.5 | 0.03 ~ 0.5 | 0.25 | 0.5 | ≤0.015 ~ 0.06 |
| Rifampicin | ≤0.015 ~ >128 | 4 | >16 | ≤0.03 ~ 16 | 16 | >16 | 0.03 ~ 16 |
| Ciprofloxacin | ≤0.125 ~ >128 | 32 | >128 | 1 ~ >128 | 128 | >128 | 0.25 ~ >128 |
| Mupirocin | 0.5 ~ >128 | >128 | >128 | 8 ~ >128 | >128 | >128 | 8 ~ >128 |
| Clindamycin | ≤0.125 ~ >128 | >128 | >128 | 4 ~ >128 | >128 | >128 | 8 ~ >128 |
| Linezolid | 8 ~ >128 | >128 | >128 | 128 ~ >128 | >128 | >128 | 64 ~ >128 |
| Oxacillin | 0.25 ~ >128 | >128 | >128 | 1 ~ >128 | 32 | >128 | 0.5 ~ >128 |
| Vancomycin | ≤0.125 ~ >128 | >128 | >128 | 1 ~ >128 | >128 | >128 | 4 ~ >128 |

The results in Table 1-2 show that the compound of formula (I) had a relatively good antibacterial activity against *staphylococcus* (including *Staphylococcus aureus* and *Staphylococcus epidermidis*) isolates, and the MIC ranges for them were ≤0.015-0.25 µg/mL, ≤0.015-0.03 µg/mL, and ≤0.015-0.03 µg/mL, respectively; the MIC₅₀ values of the compound of formula (I) for staphylococci and *Staphylococcus aureus* were both ≤0.015 µg/mL, and the compound was significantly superior to linezolid (the MIC₅₀ values were both 2 µg/mL) and vancomycin (the MIC₅₀ values were both 2 µg/mL); the MIC₉₀ values of the compound of formula (I) were both ≤0.015 µg/mL, and the compound was significantly superior to linezolid (the MIC₉₀ values were both 4 µg/mL) and vancomycin (the MIC₉₀ values were both 2 µg/mL).

The results in Tables 1-3 show that the compound of formula (I) had a relatively good antibacterial activity against *in vitro* biofilms of staphylococci (including *Staphylococcus aureus* and *Staphylococcus epidermidis*)*,* and the MBBC ranges for them were ≤0.015-1 µg/mL, 0.03-0.5 µg/mL, and ≤0.015-0.06 µg/mL, respectively; the MBBC₅₀ values of the compound of formula (I) for staphylococci and *Staphylococcus aureus* were 0.06 µg/mL and 0.25 µg/mL, respectively, and the compound was significantly superior to linezolid (the MBBC₅₀ values were both >128 µg/mL) and vancomycin (the MBBC₅₀ values were both >128 µg/mL); the MBBC₉₀ values of the compound were both 0.5 µg/mL, and the compound was significantly superior to linezolid (the MBBC₉₀ values were both >128 µg/mL) and vancomycin (the MBBC₉₀ values were both >128 µg/mL).

The above results overall show that the compound of formula (I) has a good antibacterial and bactericidal activity against Gram-positive bacteria and *Staphylococcus* (including *Staphylococcus aureus* and *Staphylococcus epidermidis*) isolates among the Gram-positive bacteria and *in vitro* biofilms, and the antibacterial and bactericidal activity is significantly superior to that of clinical first-line drugs linezolid and vancomycin. The compound has potential clinical advantages for the treatment of diabetic foot infections.

### Example 2

### Pharmacodynamic Evaluation of Treatment Method of the Present Application in Mouse Staphylococcus aureus Skin Infection Model

### 1. Experimental method

In this example, a stable mouse skin infection model was constructed using *S. aureus* NRS 384 to evaluate the antibacterial activity of the ointment comprising the compound of formula (I) described in the present application on the skin surface. In this example, a total of 6 groups were set, including a blank control group (with the experimental endpoint set at 4 hours after infection), a vehicle group, a Bactroban ointment group, and ointment groups containing low, medium, and high doses of the compound of formula (I). Each of the above groups had 8 animals, and the specific experimental settings are as follows:

| **Grouping** | **Number of animals** | **Inoculation dose** | **Treatment regimen: transdermal spreading** | **Administration time** | **Experimental endpoint** | **Detection indicator** |
|---|---|---|---|---|---|---|
| Blank control group | 8 | | N.A. | N.A. | 4 hours | |
| Vehicle group | 8 | | N.A. | 4 hours, 19 hours, 26 hours, 43 hours, 50 hours, 67 hours | 72 hours | |
| Bactroban ointment group | 8 | | 30 mg/mouse (containing 0.6 mg of mupirocin) | | | |
| 0.5% compound of formula (I) ointment group | 8 | *S. aureus* NRS 384 5.0E+07 CFU/mouse | 30 mg/mouse (containing 0.15 mg of compound of formula (I)) | | | CFU (skin) |
| 0.05% compound of formula (I) ointment group | 8 | | 30 mg/mouse (containing 0.015 mg of compound of formula (I)) | | | |
| 0.005% compound of formula (I) ointment group | 8 | | 30 mg/mouse (containing 0.0015 mg of compound of formula (I)) | | | |

### 2. Analysis of results

At 72 hours after infection, the skin samples of the mice in each treatment group were collected in a sterile environment and placed in a 15 mL centrifuge tube containing 5 mL of sterile normal saline for injection. The homogenization was performed for about 1 minute using a homogenizer. 200 µL of the homogenate was pipetted and diluted in a gradient of 10⁰-fold to 10⁻⁵-fold with sterile normal saline for injection. 10 µL of the homogenate was spotted onto a plate for each dilution gradient, and the plate was incubated in an incubator at 35±2 °C overnight, followed by CFU counting the next day.

**Table 2. Counting results of bacterial load on mouse skin**

| **Group** | **Strain** | **Treatment regimen (transdermal spreading, b.i.d.)** | **Administration time** | **Bacterial load on skin log (CFU/mouse)** |
|---|---|---|---|---|
| A | | Blank control 4 h | | 8.343±0. 132 |
| B | | Vehicle 72 h | | 9.154±0.2 73 |
| C | | 2% Bactroban ointment 30 mg/mouse | | 7.035±0. 591* |
| D | *S. aureus* NRS 384 3.85E+07 (measured) CFU/mouse | 0.5% compound of formula (I) ointment 30 mg/mouse | 4 hours, 19 hours, 26 hours, 43 hours, 50 hours, 67 hours | 4.436±0. 850*** |
| E | | 0.05% compound of formula (I) ointment 30 mg/mouse | | 6.054±1. 060*** |
| F | | 0.005% compound of formula (I) ointment 30 mg/mouse | | 8.091±0. 687 |

| | | | | |
|---|---|---|---|---|
| Note: Compared to the model group, p*** < 0.001, indicating highly significant differences; compared to the model group, 0.01 < p* < 0.05, indicating significant differences; a one-way ANOVA analysis method was used. | | | | |

The results of this pharmacodynamic experiment showed that compared to the bacterial load on the skin of the vehicle group of 9.154±0.273 log, the bacterial load on the skin of the mice treated with the Bactroban ointment (2% mupirocin) was reduced by 2.119 log, indicating that the Bactroban ointment had a good bactericidal effect; the bacterial load on the skin of the mice treated with the ointments containing 0.5%, 0.05%, and 0.005% compound of formula (I) were reduced by 4.718 log, 3.100 log, and 1.063 log, respectively. This indicates that the ointments of the high-dose group (0.5%) and the medium-dose group (0.05%) exhibited good bactericidal effects in this skin infection model, showing highly significant differences compared to the animals in the vehicle group and superior effects to the Bactroban ointment treatment group, and further demonstrating the potential clinical advantage thereof for the treatment of diabetic foot infections.

## Claims

1. A method for preventing or treating diabetic foot infections (DFI) or alleviating DFI-related disorders, comprising administering a topical therapeutic agent to a patient in need, wherein the topical therapeutic agent comprises a compound represented by formula (I) and/or a pharmaceutically acceptable salt, hydrate, solvate, prodrug, metabolite, or deuteride thereof, and the DFI is caused by Gram-positive bacteria,

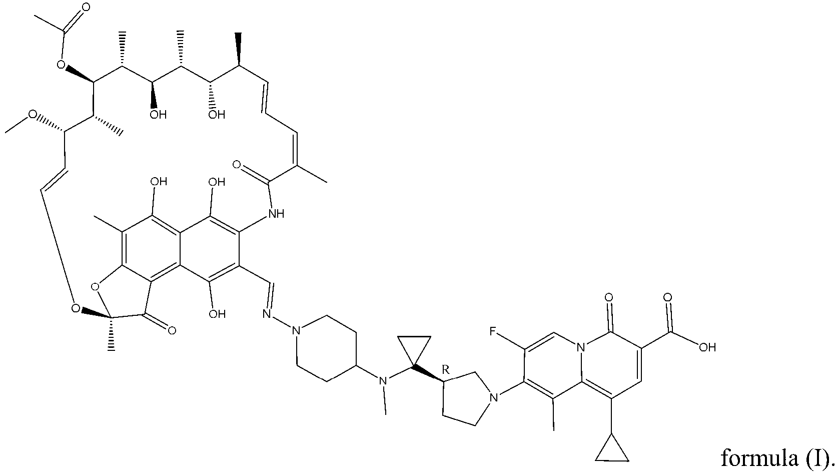

2. The method according to claim 1, wherein the Gram-positive bacteria comprise a drug-resistant strain.

3. The method according to any one of claims 1-2, wherein the topical therapeutic agent is an ointment formulation.

4. The method according to any one of claims 1-3, wherein the topical therapeutic agent further comprises one or more of polyethylene glycol 400, polyethylene glycol 3350, propylene glycol, a solubilizer, and an antioxidant.

5. The method according to any one of claims 1-4, wherein the topical therapeutic agent consists of the polyethylene glycol 400, the polyethylene glycol 3350, the propylene glycol, the solubilizer, the antioxidant, and the compound represented by formula (I) and/or the pharmaceutically acceptable salt, hydrate, solvate, prodrug, metabolite, or deuteride thereof.

6. The method according to any one of claims 1-5, wherein the compound represented by formula (I) and/or the pharmaceutically acceptable salt, hydrate, solvate, prodrug, metabolite, or deuteride thereof are used in an amount of about 0.3% to about 0.7% of the total mass of the topical therapeutic agent.

7. The method according to any one of claims 1-6, wherein the polyethylene glycol 400 is used in an amount of about 55% to about 60% of the total mass of the topical therapeutic agent.

8. The method according to any one of claims 1-7, wherein the polyethylene glycol 3350 is used in an amount of about 28% to about 33% of the total mass of the topical therapeutic agent.

9. The method according to any one of claims 1-8, wherein the propylene glycol is used in an amount of about 8% to about 12% of the total mass of the topical therapeutic agent.

10. The method according to any one of claims 1-9, wherein the solubilizer is used in an amount of about 0.1% to about 1% of the total mass.

11. The method according to any one of claims 1-10, wherein the antioxidant is used in an amount of about 0.5% to about 1% of the total mass.

12. The method according to any one of claims 1-11, wherein the topical therapeutic agent consists of the following components:
based on a total mass percentage of 100%, the compound represented by formula (I) and/or the pharmaceutically acceptable salt, hydrate, solvate, prodrug, metabolite, or deuteride thereof used in an amount of about 0.3% to about 0.7% of the total mass of the topical therapeutic agent, the polyethylene glycol 400 used in an amount of about 55% to about 60% of the total mass of the topical therapeutic agent, the polyethylene glycol 3350 used in an amount of about 28% to about 33% of the total mass of the topical therapeutic agent, the propylene glycol used in an amount of about 8% to about 12% of the total mass of the topical therapeutic agent, the solubilizer used in an amount of about 0.1% to about 1% of the total mass of the topical therapeutic agent, and the antioxidant used in an amount of about 0.5% to about 1% of the total mass of the topical therapeutic agent.

13. The method according to any one of claims 1-12, comprising administering the topical therapeutic agent to the patient in need at an administration frequency optionally selected from the following:
once every 3 days, once every 2 days, once daily, and twice daily.

14. The method according to any one of claims 1-13, wherein the diabetic foot infections are caused by *Staphylococcus aureus* and/or coagulase-negative *staphylococcus.*

15. The method according to claim 14, wherein the *Staphylococcus aureus* comprises a drug-resistant strain thereof, and the coagulase-negative *staphylococcus* comprises a drug-resistant strain thereof.

16. The method according to any one of claims 1-15, further comprising administering an effective amount of an additional therapeutic agent to the patient in need.

17. Use of the compound represented by formula (I)
and/or the pharmaceutically acceptable salt, hydrate, solvate, prodrug, metabolite, or deuteride thereof in preparing a topical therapeutic agent for preventing or treating diabetic foot infections (DFI) or alleviating DFI-related disorders, wherein the DFI is caused by Gram-positive bacteria, and the topical therapeutic agent comprises the compound represented by formula (I) and/or the pharmaceutically acceptable salt, hydrate, solvate, prodrug, metabolite, or deuteride thereof.

18. The use according to claim 17, wherein the Gram-positive bacteria comprise a drug-resistant strain thereof.

19. The use according to any one of claims 17-18, wherein the topical therapeutic agent is an ointment formulation.

20. The use according to any one of claims 17-19, wherein the topical therapeutic agent further comprises one or more of polyethylene glycol 400, polyethylene glycol 3350, propylene glycol, a solubilizer, and an antioxidant.

21. The use according to any one of claims 17-20, wherein the topical therapeutic agent consists of polyethylene glycol 400, polyethylene glycol 3350, propylene glycol, a solubilizer, an antioxidant, and the compound represented by formula (I) and/or the pharmaceutically acceptable salt, hydrate, solvate, prodrug, metabolite, or deuteride thereof.

22. The use according to any one of claims 17-21, wherein the compound represented by formula (I) and/or the pharmaceutically acceptable salt, hydrate, solvate, prodrug, metabolite, or deuteride thereof are used in an amount of about 0.3% to about 0.7% of the total mass of the topical therapeutic agent.

23. The use according to any one of claims 17-22, wherein the polyethylene glycol 400 is used in an amount of about 55% to about 60% of the total mass of the topical therapeutic agent.

24. The use according to any one of claims 17-23, wherein the polyethylene glycol 3350 is used in an amount of about 28% to about 33% of the total mass of the topical therapeutic agent.

25. The use according to any one of claims 17-24, wherein the propylene glycol is used in an amount of about 8% to about 12% of the total mass of the topical therapeutic agent.

26. The use according to any one of claims 17-25, wherein the solubilizer is used in an amount of about 0.1% to about 1% of the total mass.

27. The use according to any one of claims 17-26, wherein the antioxidant is used in an amount of about 0.5% to about 1% of the total mass.

28. The use according to any one of claims 17-27, wherein the topical therapeutic agent consists of the following components:
based on a total mass percentage of 100%, the compound represented by formula (I) and/or the pharmaceutically acceptable salt, hydrate, solvate, prodrug, metabolite, or deuteride thereof used in an amount of about 0.3% to about 0.7% of the total mass of the topical therapeutic agent, the polyethylene glycol 400 used in an amount of about 55% to about 60% of the total mass of the topical therapeutic agent, the polyethylene glycol 3350 used in an amount of about 28% to about 33% of the total mass of the topical therapeutic agent, the propylene glycol used in an amount of about 8% to about 12% of the total mass of the topical therapeutic agent, the solubilizer used in an amount of about 0.1% to about 1% of the total mass of the topical therapeutic agent, and the antioxidant used in an amount of about 0.5% to about 1% of the total mass of the topical therapeutic agent.

29. The use according to any one of claims 17-28, wherein the topical therapeutic agent is formulated to be suitable for administration to a patient in need at an administration frequency optionally selected from the following: once every 3 days, once every 2 days, once daily, and twice daily.

30. The use according to any one of claims 17-29, wherein the diabetic foot infection is caused by *Staphylococcus aureus* and/or coagulase-negative *staphylococcus.*

31. The use according to claim 30, wherein the *Staphylococcus aureus* comprises a drug-resistant strain thereof, and the coagulase-negative staphylococcus comprises a drug-resistant strain thereof.

32. The use according to any one of claims 17-31, wherein the topical therapeutic agent is formulated to be suitable for co-administration with an effective amount of an additional therapeutic agent to the patient in need.
